# FASCICULE DE BREVET EUROPEEN

(11) **EP 3 080 276 B1**
(45) Date de publication et mention de la délivrance du brevet: **07.03.2018**
(21) Numéro de dépôt: 14830614.5
(22) Date de dépôt: 09.12.2014
(51) Int. Cl.: C12N 15/82

(54) **POLYPEPTIDES ENTOMOTOXIQUES**
ENTOMOTOXISCHE POLYPEPTIDE
ENTOMOTOXIC POLYPEPTIDES

(30) Priorité: 10.12.2013 FR 1362361
(43) Date de publication de la demande: 19.10.2016
(73) Titulaire: Institut National De La Recherche Agronomique, 75007 Paris (FR); Institut National des Sciences Appliquées de Lyon, 69621 Villeurbanne (FR)
(72) Inventeur: ROYER, Corinne, F-69110 Sainte Foy Les Lyon (FR); DA SILVA, Pedro, F-69003 Lyon (FR); GRESSENT, Frédéric, F-34730 PRADES-LE-LEZ (FR); KARAKI, Lamis, MarElias , Beyrouth (LB); RAHBE, Yvan, F-69100 Villeurbanne (FR)
(74) Mandataire: Cabinet Becker et Associés
(86) Numéro de dépôt international: PCT/IB2014/066727
(87) Numéro de publication internationale: WO 2015/087238

(56) Documents cités:
- WO-A1-99/58695
- WO-A1-2009/056689
- US-A1- 2012 244 575
- LOUIS ET AL: "Broad screening of the legume family for variability in seed insecticidal activities and for the occurrence of the A1b-like knottin peptide entomotoxins", PHYTOCHEMISTRY, PERGAMON PRESS, GB, vol. 68, no. 4, 3 février 2007 (2007-02-03), pages 521-535, XP005889403, ISSN: 0031-9422, DOI: 10.1016/J.PHYTOCHEM.2006.11.032

## Description

La présente invention concerne des polypeptides entomotoxiques de la famille des albumines 1b de légumineuses et leurs utilisations.

Les albumines 1b (A1b) sont des peptides entomotoxiques de la famille des knottines. Ces peptides ont été initialement identifiées dans les graines de pois (HIGGINS et al., J. Biol. Chem., 261, 11124-30, 1986), sous la dénomination de PA1b (pour Pea Albumin 1 subunit b), puis chez le soja, où elles sont également dénommées leginsuline (WATANABE et al., Eur. J. Biochem., 224, 167-72, 1994), et ultérieurement, dans les graines d'autres Fabacées appartenant notamment aux genres *Vicia, Phaseolus,* et *Glycine* (LOUIS et al., Plant. Sci., 167, 705-14, 2004; LOUIS et al., Phytochemistry, 68, 521-35, 2007).

Les séquences des peptides A1b sont fortement conservées ; elles comprennent notamment 11 résidus invariants : 5 résidus proline, et 6 résidus cystéine formant 3 ponts disulfure. La structure tertiaire de PA1b (JOUVENSAL et al., Biochemistry 42, 11915-23, 2003) comprend un noeud formé par les trois ponts disulfures, trois feuillets β antiparallèles, une boucle L1 contenant la séquence conservée CSPFE, et une boucle L2 dont l'hydrophobicité des acides aminés est conservée.

Il a été observé que plusieurs isoformes d'A1b peuvent coexister dans une même plante (Demande PCT WO 99/58695 ; TAYLOR et al., J. Agric. Food. Chem., 52, 7499-506, 2004 ; TAYLOR et al., J. Agric. Food. Chem., 52, 7491-8, 2004), ce qui indique que ces peptides appartiennent à une famille multigénique dont les membres ont divergé légèrement.

A1b provient de la maturation d'une polyprotéine dénommée A1 (pour « Albumin 1 »). A1 est composée, de l'extrémité N-terminale vers l'extrémité C-terminale, d'un peptide signal, de la sous-unité b (A1b) et de son propeptide, de la sous-unité a (A1a) et de son propeptide. Après clivage endopeptidique du peptide signal, la proprotéine est adressée dans les corps protéiques de stockage de la graine. Dans ces structures dérivées de vacuoles, les propeptides sont éliminés par des endopeptidases, libérant ainsi deux protéines matures : A1b et A1a.

La Demande PCT WO 99/58695 décrit la mise en évidence de l'activité entomotoxique de PA1b, et son utilisation comme insecticide, en particulier pour protéger des graines de céréales vis-à vis des charançons des grains. Ultérieurement, il a été rapporté que d'autres insectes étaient sensibles à PA1b: s'agit en particulier de coléoptères (*Sitophilus sp, Harmonia axyridis*)*,* de certains diptères, et notamment des moustiques (*Culex pipiens* et *Aedes aegyptii*), et de certaines espèces de pucerons telles qu'*Acyrthosiphon pisum* ; chez les lépidoptères, *Mamestra brassicae, Spodoptera frugiperda,* et *Ostrinia nubilalis* sont insensibles à PA1b, alors qu'*Ephestia khuniella* et les cellules Sf9 (originaires de *Spodoptera frugiperda*), y sont sensibles (GRESSENT et al., J Insect Sci, 7, 1-10, 2007 ; GRESSENT et al., Toxins (Base1), 3, 1502-17, 2011). Il a été montré récemment que l'activité insecticide de PA1b faisait intervenir l'inhibition d'une pompe à proton membranaire, la V-ATPase, dont l'activité au niveau de l'intestin est essentielle chez les insectes, car elle fournit l'énergie nécessaire à l'absorption des nutriments (CHOUABE et al., J Biol Chem, 286, 36291-6, 2011).

Pour déterminer les résidus essentiels à l'activité entomotoxique de PA1b, DA SILVA et al. (DA SILVA et al., J Biol Chem, 285, 32689-94, 2010) ont effectué différentes mutations ponctuelles dans la séquence de la protéine, notamment au niveau des boucles L1 et L2. Ils ont ainsi montré que la présence des résidus phénylalanine en position 10, arginine en position 21, isoleucine en position 23, et leucine en position 27, était essentielle au maintien de l'activité toxique.

Les albumines A1b sont parmi les rares peptides entomotoxiques actifs par voie orale connus à l'heure actuelle. Elles présentent *a priori,* par rapport aux pesticides chimiques, de nombreux avantages, notamment pour la préservation de la qualité des sols et des eaux après traitement ainsi que pour la protection de l'agriculteur (lors du traitement) et du consommateur.

En outre certains des insectes sensibles à A1b ont un impact économique ou sanitaire très important. Par exemple, les charançons des céréales sont responsables de pertes céréalières approchant les 20% dans le monde, les moustiques sont les premiers vecteurs de maladies humaines et des mammifères dans le monde et les pucerons sont des vecteurs de virus des plantes.

Actuellement, les albumines A1b sont principalement obtenues par extraction à partir des graines de légumineuses (Demande PCT WO 99/58695), ou par synthèse peptidique, suivie de repliement *in vitro* (DA SILVA et al., Biopolymers, 92, 436-44, 2009). Elles ont également été produites sous forme recombinante, par exemple chez *E.coli,* comme décrit notamment par (HANADA & HIRANO, Biochemistry, 43, 12105-12, 2004) dans le cas de la leginsuline, homologue de PA1b chez le soja, ou chez la levure *Pichia pastoris* (Demandes CN101082046 et CN101033465).

PA1b a également été exprimé dans des plantes transgéniques, notamment dans des céréales, afin de les protéger contre les charançons : ainsi, des plants de riz transgénique exprimant la protéine PA1b de pois ont été obtenus (PETIT, Thèse de doctorat, Université Montpellier II, 2006 ; Demande PCT WO 2009/056689), et il a été observé que l'accumulation de PA1b dans les graines issues de ces plants leur conférait des propriétés entomotoxiques vis-à-vis des larves et des adultes de *S. oryzae.* Cependant, le niveau de toxicité obtenu n'était pas suffisant pour permettre une protection complète.

Les Inventeurs ont entrepris de rechercher s'il existait chez d'autres légumineuses, des peptides A1b dotés de propriétés entomotoxiques supérieures à celles de la protéine PA1b de référence du pois.

Cette recherche leur a permis d'identifier, chez la luzerne (*Medicago truncatula*)*,* une albumine A1b qui est environ 10 fois plus active que l'albumine PA1b de pois.

Cette albumine A1b est un polypeptide de 41 acides aminés répondant à la séquence suivante :
ASCPNVGAVCSPFETKPCGNVKDCRCLPWGLFFGTCINPTG (SEQ ID NO: 1), qui représente la forme mature d'une albumine A1b.

Elle est issue de la maturation d'une polyprotéine A1 dont la séquence est la suivante :

L'alignement de séquences du polypeptide PA1b de *Pisum sativum* (SEQ ID NO: 3) avec le polypeptide A1b de séquence SEQ ID NO: 1 de la présente invention est représenté ci-dessous.

| | |
|---|---|
| PA1b : | ASC-N-G-VCSP**F**EMPPCG-TSAC**R**C**I**PVG**L**VIGYCRNPSG |
| SEQ ID NO: 1 : | ASCPNVGAVCSP**F**ETKPCGNVKDC**R**CLPWG**L**FFGTCINPTG |

Les variations d'acides aminés sont soulignées ; les résidus identifiés comme essentiels à l'activité entomotoxique chez PA1b (DA SILVA et al., 2010, précité) sont indiqués en gras.

La présente invention a pour objet le polypeptide A1b de séquence SEQ ID NO: 1.

Elle englobe également des polypeptides A1b dérivés du peptide SEQ ID NO: 1 par des substitutions d'acides aminés conservant le profil d'hydrophobicité, la structure tertiaire, et les propriétés entomotoxiques du polypeptide SEQ ID NO: 1.

Des exemples non-limitatifs de substitutions sont indiqués dans le Tableau I ci-dessous. Les propriétés entomotoxiques des peptides substitués peuvent être facilement vérifiées par exemple en déterminant leur affinité pour le site de liaison à PA1b, et/ou leur toxicité sur des cultures de cellules Sf9 de *Spodoptera frugiperda,* comme décrit plus loin dans les Exemples.

**Tableau I**

| Position | Résidu | Exemples de substituants | Substituants préférés |
|---|---|---|---|
| 1 | A | | |
| 2 | S | T | |
| 3 | C | | |
| 4 | P | | |
| 5 | N | Q | |
| 6 | V | L, I, W, F | L, I, F |
| 7 | G | | |
| 8 | A | | |
| 9 | V | L, I, W, F | L, I, F |
| 10 | C | | |
| 11 | S | T | |
| 12 | P | | |
| 13 | F | V, L, I, W | V, L, I |
| 14 | E | D | |
| 15 | T | S | |
| 16 | K | R | |
| 17 | P | | |
| 18 | C | | |
| 19 | G | | |
| 20 | N | Q | |
| 21 | V | L, I, W, F | L, I, F |
| 22 | K | R | |
| 23 | D | E | |
| 24 | C | | |
| 25 | R | K | |
| 26 | C | | |
| 27 | L | V, I, W, F | V, I, F |
| 28 | P | | |
| 29 | W | V, L, I, F | |
| 30 | G | | |
| 31 | L | V, I, W, F | V, I, F |
| 32 | F | V, L, I, W | V, L, I |
| 33 | F | V, L, I, W | V, L, I |
| 34 | G | | |
| 35 | T | S | |
| 36 | C | | |
| 37 | I | V, L, W,F | V, L, F |
| 38 | N | Q | |
| 39 | P | | |
| 40 | T | S | |
| 41 | G | | |

Un polypeptide conforme à l'invention peut être obtenu par les méthodes connues en elles-mêmes, précédemment utilisées pour d'autres polypeptides A1b.

Il peut notamment être produit par synthèse peptidique, ou bien par génie génétique, en exprimant un polynucléotide codant pour ce polypeptide dans une cellule ou un organisme hôte approprié.

La présente invention englobe également des cassettes d'expression recombinantes comprenant un polynucléotide contenant une séquence codant pour un polypeptide conforme à l'invention, sous contrôle transcriptionnel d'un promoteur approprié.

Dans les cassettes d'expression conformes à l'invention, la séquence codant pour un polypeptide conforme à l'invention, tel que le polypeptide SEQ ID NO: 1, peut être utilisée sous forme isolée, c'est-à-dire qu'elle n'est pas associée à la séquence codant pour le propeptide correspondant, ni aux séquences codant pour la sous-unité A1a et son propeptide. Alternativement, lorsque cette séquence codante est destinée à être exprimé dans des cellules-hôtes pouvant effectuer la maturation d'un polypeptide A1b, on peut lui associer le cas échéant la séquence codant pour son propeptide, et en outre, éventuellement la séquence codant pour la sous-unité A1a et/ou la séquence codant pour le propeptide de ladite sous-unités A1a.

Dans tous les cas, la séquence codant pour le polypeptide conforme à l'invention peut également être associée à un peptide signal, qui peut être le peptide signal endogène d'une polyprotéine A1, ou le cas échéant, un peptide signal hétérologue.

Pour la construction des cassettes d'expression conformes à l'invention le choix du promoteur approprié sera effectué de manière classique par l'homme du métier, en fonction notamment de la cellule ou de l'organisme hôte choisi pour l'expression. Il peut ainsi s'agir d'un promoteur procaryote, ou d'un promoteur eucaryote. Ce promoteur peut être constitutif, ou bien inductible si l'on souhaite exprimer préférentiellement le polypeptide d'intérêt dans certaines conditions environnementales ; également, en cas d'expression dans un organisme hôte, on peut utiliser un promoteur tissu-spécifique, permettant une expression préférentielle dans certains tissus ou organes cibles.

Les cassettes d'expression conformes à l'invention peuvent comprendre en outre d'autres éléments usuellement employés dans ce type de constructions pour améliorer l'expression du gène d'intérêt, tels qu'un terminateur de transcription, des séquences « enhancer », des introns, etc.

La présente invention a également pour objet un vecteur recombinant, résultant de l'insertion d'une cassette d'expression conforme à l'invention dans un vecteur hôte.

Parmi la très grande variété de vecteurs-hôtes disponibles, le choix du vecteur le plus approprié sera effectué, de manière classique, par l'homme du métier en fonction notamment de critères tels que la cellule ou l'organisme hôte choisi, le protocole de transformation envisagé, etc.

La présente invention englobe également des cellules et organismes hôtes transformés par une cassette d'expression conforme à l'invention.

On entend ici par cellule ou organisme transformé par une cassette d'expression, toute cellule ou organisme hôte dont le contenu génétique a été modifié par transfert de ladite cassette d'expression dans ladite cellule ou ledit organisme, quelle que soit la méthode de transfert qui a été utilisée, et que l'information génétique apportée par ladite cassette soit intégrée dans l'ADN chromosomique ou qu'elle demeure extra-chromosomique.

Les cellules transformées conformes à l'invention peuvent être des cellules procaryotes, ou eucaryotes. Dans le cas de cellules procaryotes, il peut par exemple s'agir d'*E*. *coli,* d'Agrobactéries telles qu'*Agrobacterium tumefaciens ou Agrobacterium rhizogenes,* ou de bactéries entomopathogènes ou symbiotiques d'insectes, notamment de bactéries capables de coloniser le tube digestif des insectes. Dans le cas de cellules eucaryotes, il peut s'agir notamment de cellules végétales, qui peuvent être issues de plantes dicotylédones ou monocotylédones, de cellules de levure, ou de cellules d'insecte. Des organismes transformés conformes à l'invention peuvent être notamment des plantes transgéniques.

La présente invention a également pour objet l'utilisation d'un polypeptide conforme à l'invention comme insecticide. Les insectes concernés sont notamment ceux sensibles à PA1b (cf. GRESSENT et al., 2007, 2011, cités ci-dessus). En outre, l'homme du métier peut aisément évaluer la sensibilité d'autres insectes au polypeptide A1b conforme à l'invention, en ajoutant ce polypeptide, à différentes doses, à l'alimentation des insectes à tester.

Dans le cadre de cette utilisation comme insecticide, le polypeptide conforme à l'invention peut être utilisé comme décrit dans la Demande PCT WO 99/58695.

De manière avantageuse il peut être exprimé dans une plante transgénique, afin de protéger ladite plante contre les insectes ravageurs.

La présente invention a également pour objet un procédé pour obtenir une plante transgénique exprimant un polypeptide conforme à l'invention, caractérisé en ce qu'elle comprend les étapes suivantes :
- la transformation de cellules végétales par une cassette d'expression conforme à l'invention ;
- la régénération de plantes à partir des cellules transformées ;
- la sélection des plantes ayant intégré dans leur génome ladite cassette d'expression.

Pour la mise en oeuvre de la présente invention, des techniques très nombreuses de transformation de cellules végétales germinales ou somatiques, (isolées, sous forme de cultures de tissus ou d'organe, ou sur la plante entière), et de régénération des plantes sont disponibles. Le choix de la méthode la plus appropriée dépend généralement de la plante concernée.

A titre d'exemples non-limitatifs, on citera la transformation de protoplastes en présence de polyéthylène glycol, l'électroporation, l'utilisation d'un canon à particules, la micro-injection cytoplasmique ou nucléaire, ou la transformation par l'intermédiaire d'*Agrobacterium.* Dans le cas des plantes monocotylédones on utilisera préférentiellement la transformation par *Agrobacterium tumefaciens.*

La présente invention a également pour objet une plante transgénique comprenant dans son génome au moins une copie d'une cassette d'expression conforme à l'invention.

On définit ici comme plante transgénique une plante transformée chez laquelle l'information génétique exogène apportée par un polynucléotide transformant est intégrée de manière stable dans l'ADN chromosomique, sous forme de transgène, et peut ainsi être transmise aux descendants de ladite plante. Cette définition englobe donc également les descendants des plantes résultant de la transgénèse initiale, dès lors qu'ils contiennent dans leur génome une copie du transgène.

Les plantes transgéniques conformes à l'invention expriment un polypeptide A1b conforme à l'invention dans la plante entière, ou au moins dans certains tissus ou organes de celle-ci, par exemple les graines. Cette expression confère aux tissus et organes concernés une toxicité vis-à-vis d'insectes ravageurs, et augmente donc leur résistance aux attaques de ces insectes.

Pour accroître leur spectre de résistance vis-à-vis des insectes, des plantes transgéniques conformes à l'invention peuvent également comprendre, le cas échéant, un ou plusieurs autre(s) gènes codant pour une ou plusieurs autre(s) entomotoxines. A titre d'exemples on citera les toxines Cry3 de *Bacillus thuringiensis,* notamment Cry3A, des inhibiteurs de protéases, des toxines Vip, l'avidine, des lectines, etc.

La présente invention s'applique à toutes les plantes qui n'expriment pas naturellement un polypeptide A1b conforme à l'invention, ou bien qui ne l'expriment pas naturellement dans un organe que l'on souhaite protéger. Elle présente un intérêt particulier chez les céréales, telles que le blé, le maïs ou le riz, mais peut également être utilisée chez toute autre plante susceptible d'être attaquée par des insectes ravageurs sensibles à cette protéine. Ceci peut inclure notamment des légumineuses autres que *Medicago truncatula,* dont elle peut renforcer la résistance naturelle : à titre d'exemples, on citera le pois, le haricot, le soja, etc.

Le matériel végétal (protoplastes, cals, boutures, semences, etc.) obtenu à partir des cellules transformées ou des plantes transgéniques conformes à l'invention fait également partie de l'objet de la présente invention. L'invention englobe également les produits obtenus à partir des plantes transgéniques conformes à l'invention, et dans lesquels un polypeptide A1b conforme à l'invention est présent ; il s'agit notamment des graines et de leurs produits dérivés, par exemple farines ou semoules.

Dans le cadre de l'utilisation comme insecticide, le polypeptide A1b conforme à l'invention peut également être exprimé dans un micro-organisme entomopathogène ou non, capable d'infecter l'insecte que l'on veut cibler. Il peut s'agir par exemple de bactéries (DURVASULA et al., Proc Natl Acad Sci USA, 94, 3274-8, 1997; RIEHLE et al., Int J Parasitol, 37, 595-603, 2007; DURVASULA et al., Exp Parasitol, 119, 94-8, 2008) ou de virus, tels que des baculovirus (BONNING & NUSAWARDANI, Methods Mol Biol, 388, 359-66, 2007 ; INCEOGLU et al., Adv Virus Res, 68, 323-60, 2006) ou des densovirus (REN et al., PLoS Pathog, 4, e1000135, 2008).

Ce mode de mise en oeuvre présente un intérêt tout particulier dans le cas de l'utilisation vis-à-vis d'insectes non phytophages, tels que les moustiques.

Les bactéries et virus recombinants contenant une cassette d'expression conforme à l'invention, et l'utilisation de ces bactéries et virus comme insecticide font également partie de l'objet de la présente invention.

La production de baculovirus ou densovirus recombinants conformes à l'invention peut être effectuée par des méthodes standard, connues en elles-mêmes (cf. par exemple dans le cas des baculovirus : O'REILLY et al., BACULOVIRUS EXPRESSION VECTORS : A LABORATORY MANUAL, Freeman and Cie, New York, 1994, et dans celui des densovirus : PCT WO 93/01295 ; PCT WO 96/14423 ; BOSSIN et al., Journal of Virology, 77, 11060-71, 2003; CARLSON et al., Advances in Virus Research, Volume 68, 361-92, 2006)

Dans le cas de virus produits dans des cellules d'insectes sensibles au polypeptide A1b conforme à l'invention (par exemple des baculovirus produits dans des cellules d'insecte Sf9), le promoteur utilisé dans la cassette d'expression pour contrôler l'expression du polypeptide A1b sera de préférence un promoteur tardif (par exemple le promoteur de la polyédrine ou le promoteur p10), pour permettre de limiter les effets toxiques de ce polypeptide sur les cellules d'insecte utilisées pour la production de ces baculovirus.

La présente invention sera mieux comprise à l'aide du complément de description qui va suivre, qui se réfère à un exemple non-limitatif illustrant les propriétés du polypeptide A1b conforme à l'invention.

### EXEMPLE 1 : IDENTIFICATION ET CARACTÉRISATION D'ALBUMINES A1B ENTOMOTOXIQUES CHEZ MEDICAGO TRUNCATULA

Une analyse *in silico* des données génomiques et ESTs de *Medicago truncatula* a permis d'identifier 53 gènes de la famille des albumines A1. 43 de ces gènes contenaient la séquence complète d'une sous-unité A1b.

6 de ces gènes ont été choisis, et les polypeptides A1b correspondants ont été synthétisés chimiquement et repliés *in vitro,* comme décrit précédemment par DA SILVA et al. (Biopolymers, 92, 436-44, 2009), afin de tester leur activité. Le polypeptide PA1b de *Pisum sativum* a également été synthétisé de la même manière, pour être utilisé à titre de témoin positif.

Les séquences alignées des polypeptides synthétisés sont indiquées dans le Tableau II ci-dessous. Pour les polypeptides de *Medicago truncatula* la dénomination arbitraire utilisée ici se réfère au premier et au dernier acide aminé de la séquence, suivis du nombre d'acides aminés dans celle-ci.

**Tableau II**

| Nom | Séquence | SEQ ID NO: |
|---|---|---|
| PA1b | ASC-N-G-VCSPFEMPP-CG-TSACRCIPVGLVIGY---CRNPSG | 3 |
| AS37 | ADC-S-G-ICSPFEMPP-CR-SSDCRCIPIALIGGF---CINPIS | 4 |
| AG41 | ASCPNVGAVCSPFETKP-CGNVKDCRCLPWGLFFGT---CINPTG | 1 |
| DS37 | DEC-W-G-PCSVLQTPP-CPLSK-CYCIPLFLVVGY---CSHASS | 5 |
| QT41 | QSC-I-G-FCSVFDSKPLCGSSR-CRCNKPLNNPFVGI-CERRPST | 6 |
| GL44 | GQCARVGMRCSRALPNP-CGDIVTCRCVHLHLVGST---CIDYTGDGL | 7 |
| EG41 | EFCSSVGSFCSPFNTNP-CGYLGNCRCVPYYLYGGT---CENPFG | 8 |
| AS40 | AKC---GEACDTQFNF--CNAGDGCRCFITDAYLTLPGFCAQLST | 9 |

Ces peptides ont ensuite été testés pour leur affinité pour le site de liaison à PA1b, et pour leurs propriétés entomotoxiques.

L'affinité pour le site de liaison à PA1b a été déterminée par des essais de liaison du ligand en utilisant une toxine marquée à l'iode ¹²⁵I, comme décrit précédemment (GRESSENT et al., Eur J Biochem, 270, 2429-35, 2003).

Les propriétés entomototoxiques ont été déterminées par évaluation de la CL50 (concentration létale 50%) sur des cultures de cellules Sf9 de *Spodoptera frugiperda,* comme décrit par RAHIOUI et al. (Biochimie, 89, 1539-43, 2007) Les cellules ont été cultivées à 27° C dans du milieu de LONZA supplémenté avec 5% de sérum de veau foetal (FBS) et de 0,1% de gentamicine, puis ensemencées dans des plaques à 96 puits, 24 h avant les expériences (15 000 cellules/puits) et exposées à des concentrations croissantes de peptide synthétique. Après 24 h, la viabilité cellulaire a été déterminée en utilisant le test de viabilité CELLTITER-BLUE (PROMEGA), conformément aux instructions du fabricant. Après l'ajout du colorant, les cellules ont été incubées à 27° C pendant 4 h. L'absorbance a ensuite été mesurée à 570 et 600 nm en utilisant un lecteur de microplaques (MR 7000, DYNATECH LABORATORIES Inc., USA).

Les résultats sont indiqués dans le Tableau III ci-dessous : « - » représente un résultat négatif (pas de toxicité, ou pas de liaison dans la gamme des concentrations testées)

**Tableau III**

| Peptide | Ki (nM) | CL50 (nM) |
|---|---|---|
| PA1b | 17 ± 1,1 | 79,8 ± 3,6 |
| AS37 | 15 ± 2 | 107 ± 3,2 |
| AG41 | 1,3 ± 0,6 | 5,44 ± 1,7 |
| DS37 | 694 ± 2 | - |
| QT41 | - | - |
| GL44 | - | - |
| EG41 | 72,8 ± 2,7 | 75 ± 3,1 |
| AS40 | 10,3 ± 2 | 47 ± 2,2 |

Ces résultats montrent que certains des peptides testés (AS37, EG41, AS40) possèdent une toxicité comparable à celle du peptide PA1b de référence, tandis que d'autres (DS37, QT41, et GL44) n'ont pas de propriétés entomotoxiques. Notamment, la présence d'une tyrosine (Y) à la place de l'arginine (R) dans le motif conservé CXC (DS37 vs AS37) apparaît corrélée à une perte de propriétés de liaison et de la toxicité.

Le peptide AG41 présente une toxicité très élevée, près de dix fois supérieure à celle de PA1b.

Pour la plupart des peptides (à l'exception d'EG41) la toxicité apparaît corrélée à l'affinité pour le site de liaison à PA1b.

## Revendications

1. Polypeptide entomotoxique isolé, **caractérisé en ce qu'**il est défini par la séquence suivante :
ASCPNVGAVCSPFETKPCGNVKDCRCLPWGLFFGTCINPTG (SEQ ID NO: 1).

2. Cassette d'expression recombinante comprenant un polynucléotide contenant une séquence codant pour le polypeptide selon la revendication 1, ledit polynucléotide étant placé sous contrôle transcriptionnel d'un promoteur approprié.

3. Vecteur recombinant contenant une cassette d'expression selon la revendication 2.

4. Cellule-hôte transformée par une cassette d'expression selon la revendication 2.

5. Plante transgénique comprenant un transgène contenant une cassette d'expression selon la revendication 2.

6. Virus recombinant, contenant une cassette d'expression selon la revendication 2.

7. Utilisation d'un polypeptide selon la revendication 1, ou d'un virus recombinant selon la revendication 6 comme insecticide.

## Patentansprüche

1. Isoliertes entomotoxisches Polypeptid, **dadurch gekennzeichnet, dass** es durch die folgende Sequenz definiert ist:
ASCPNVGAVCSPFETKPCGNVKDCRCLPWGLFFGTCINPTG (SEQ ID NO: 1)

2. Rekombinante Expressionskassette, umfassend ein Polynukleotid, enthaltend eine für das Polypeptid gemäß Anspruch 1 codierende Sequenz, wobei besagtes Polynukleotid unter transkriptioneller Kontrolle eines geeigneten Promotors steht.

3. Rekombinanter Vektor, enthaltend eine Expressionskassette gemäß Anspruch 2.

4. Wirtszelle, transformiert mit einer Expressionskassette gemäß Anspruch 2.

5. Transgene Pflanze, umfassend ein Transgen, enthaltend eine Expressionskassette gemäß Anspruch 2.

6. Rekombinantes Virus, enthaltend eine Expressionskassette gemäß Anspruch 2.

7. Verwendung eines Polypeptids gemäß Anspruch 1 oder eines rekombinanten Virus gemäß Anspruch 6 als Insektizid.

## Claims

1. An isolated entomotoxic polypeptide, **characterized in that** it is defined by the following sequence:
ASCPNVGAVCSPFETKPCGNVKDCRCLPWGLFFGTCINPTG (SEQ ID NO: 1).

2. A recombinant expression cassette comprising a polynucleotide containing a sequence encoding the polypeptide as claimed in claim 1, said polynucleotide being placed under the transcriptional control of an appropriate promoter.

3. A recombinant vector containing an expression cassette as claimed in claim 2.

4. A host cell transformed with an expression cassette as claimed in claim 2.

5. A transgenic plant comprising a transgene containing an expression cassette as claimed in claim 2.

6. A recombinant virus containing an expression cassette as claimed in claim 2.

7. The use of a polypeptide as claimed in claim 1, or of a recombinant virus as claimed in claim 6, as an insecticide.


### SEQUENCE LISTING

<110> INSTITUT NATIONAL DE LA RECHERCHE AGRONOMIQUE
   INSTITUT NATIONAL DES SCIENCES APPLIQUEES DE LYON
   ROYER, Corinne
   DA SILVA, Pedro
   GRESSENT, Frédéric
   KARAKI, Lamis
   RAHBE, Yvan
<120> POLYPEPTIDES ENTOMOTOXIQUES
<130> EMT/11-F539-167-PCT
<150> FR 13 62361
   <151> 2013-12-10
<160> 9
<170> PatentIn version 3.5
<210> 1
   <211> 41
   <212> PRT
   <213> Medicago truncatula
<400> 1
   Ala Ser Cys Pro Asn Val Gly Ala Val Cys Ser Pro Phe Glu Thr Lys
   1               5                   10                  15
   Pro Cys Gly Asn Val Lys Asp Cys Arg Cys Leu Pro Trp Gly Leu Phe
               20                  25                  30
   Phe Gly Thr Cys Ile Asn Pro Thr Gly
           35                  40
<210> 2
   <211> 154
   <212> PRT
   <213> Medicago truncatula
<220>
   <221> SIGNAL
   <222> (1)..(27)
<220>
   <221> MISC_FEATURE
   <222> (28)..(68)
   <223> A1b mature
<220>
   <221> PROPEP
   <222> (69)..(76)
   <223> propeptide A1b
<220>
   <221> MISC_FEATURE
   <222> (77)..(142)
   <223> A1a mature
<220>
   <221> PROPEP
   <222> (143)..(154)
   <223> propeptide A1a
<400> 2
   Met Thr Tyr Val Lys Leu Ala Ile Leu Ala Val Leu His Leu Thr Ile
   1               5                   10                  15
   Phe Leu Ile Phe Gln Thr Lys Asn Val Glu Ala Ala Ser Cys Pro Asn
               20                  25                  30
   Val Gly Ala Val Cys Ser Pro Phe Glu Thr Lys Pro Cys Gly Asn Val
           35                  40                  45
   Lys Asp Cys Arg Cys Leu Pro Trp Gly Leu Phe Phe Gly Thr Cys Ile
       50                  55                  60
   Asn Pro Thr Gly Ser Lys Tyr Asn Met Lys Met Ile Glu Glu His Pro
   65                  70                  75                  80
   Asn Leu Cys Gln Thr His Gly Glu Cys Ile Lys Lys Gly Ser Gly Asn
                   85                  90                  95
   Phe Cys Ala Arg Tyr Ala Asn Ala Asp Ile Glu Tyr Gly Trp Cys Phe
               100                 105                 110
   Val Ser Val Ser Glu Ala Glu Arg Tyr Phe Lys Ile Gly Ser Asn Thr
           115                 120                 125
   Ala Val Lys Ser Phe Phe Lys Ile Ala Ser Lys Glu Lys Asp Tyr Leu
       130                 135                 140
   Lys Met Ala Leu Glu Ile Ala Thr Glu Glu
   145                 150
<210> 3
   <211> 37
   <212> PRT
   <213> Pisum sativum
<400> 3
   Ala Ser Cys Asn Gly Val Cys Ser Pro Phe Glu Met Pro Pro Cys Gly
   1 5 10 15
   Thr Ser Ala Cys Arg Cys Ile Pro Val Gly Leu Val Ile Gly Tyr Cys
   20 25 30
   Arg Asn Pro Ser Gly
   35
<210> 4
   <211> 37
   <212> PRT
   <213> Medicago truncatula
<400> 4
   Ala Asp Cys Ser Gly Ile Cys Ser Pro Phe Glu Met Pro Pro Cys Arg
   1 5 10 15
   Ser Ser Asp Cys Arg Cys Ile Pro Ile Ala Leu Ile Gly Gly Phe Cys
   20 25 30
   Ile Asn Pro Ile Ser
   35
<210> 5
   <211> 37
   <212> PRT
   <213> Medicago truncatula
<400> 5
   Asp Glu Cys Trp Gly Pro Cys Ser Val Leu Gln Thr Pro Pro Cys Pro
   1 5 10 15
   Leu Ser Lys Cys Tyr Cys Ile Pro Leu Phe Leu Val Val Gly Tyr Cys
   20 25 30
   Ser His Ala Ser Ser
   35
<210> 6
   <211> 41
   <212> PRT
   <213> Medicago truncatula
<400> 6
   Gln Ser Cys Ile Gly Phe Cys Ser Val Phe Asp Ser Lys Pro Leu Cys
   1               5                   10                  15
   Gly Ser Ser Arg Cys Arg Cys Asn Lys Pro Leu Asn Asn Pro Phe Val
               20                  25                  30
   Gly Ile Cys Glu Arg Arg Pro Ser Thr
           35                  40
<210> 7
   <211> 44
   <212> PRT
   <213> Medicago truncatula
<400> 7
   Gly Gln Cys Ala Arg Val Gly Met Arg Cys Ser Arg Ala Leu Pro Asn
   1               5                   10                  15
   Pro Cys Gly Asp Ile Val Thr Cys Arg Cys Val His Leu His Leu Val
               20                  25                  30
   Gly Ser Thr Cys Ile Asp Tyr Thr Gly Asp Gly Leu
           35                  40
<210> 8
   <211> 41
   <212> PRT
   <213> Medicago truncatula
<400> 8
   Glu Phe Cys Ser Ser Val Gly Ser Phe Cys Ser Pro Phe Asn Thr Asn
   1               5                   10                  15
   Pro Cys Gly Tyr Leu Gly Asn Cys Arg Cys Val Pro Tyr Tyr Leu Tyr
               20                  25                  30
   Gly Gly Thr Cys Glu Asn Pro Phe Gly
           35                  40
<210> 9
   <211> 40
   <212> PRT
   <213> Medicago truncatula
<400> 9
   Ala Lys Cys Gly Glu Ala Cys Asp Thr Gln Phe Asn Phe Cys Asn Ala
   1               5                   10                  15
   Gly Asp Gly Cys Arg Cys Phe Ile Thr Asp Ala Tyr Leu Thr Leu Pro
               20                  25                  30
   Gly Phe Cys Ala Gln Leu Ser Thr
           35                  40